# EUROPÄISCHE PATENTSCHRIFT

(11) **EP 0 293 818 B1**
(45) Veröffentlichungstag und Bekanntmachung des Hinweises auf die Patenterteilung: **24.03.1993**
(21) Anmeldenummer: 88108666.4
(22) Anmeldetag: 31.05.1988
(51) Int. Cl.: C07C 45/51, C07C 47/19

(54) **Verfahren zur Herstellung von Carbonylverbindungen durch Isomerisierung von Allylalkoholen**
Process for the preparation of carbonyl compounds by isomerization of allylalcohols
Procédé pour la préparation de dérivés carbonylés par isomérisation d'alcools allyliques

(30) Priorität: 05.06.1987 DE 3718897
(43) Veröffentlichungstag der Anmeldung: 07.12.1988
(73) Patentinhaber: BASF Aktiengesellschaft, 67063 Ludwigshafen (DE)
(72) Erfinder: Kummer, Rudolf, Dr., D-6710 Frankenthal (DE); Bertleff, Werner, Dr., D-6806 Viernheim (DE); Roeper, Michael, Dr., D-6706 Wachenheim (DE)

(56) Entgegenhaltungen:
- J. OF ORGANOMETALLIC CHEMISTRY, Band 297, 1985, Seiten C37-C40; K. FELFÖLDI et al.: "Transformation of organic compounds in the presence of metal complexes. I. Transformations of unsaturated alcohols with metal complex catalysts"
- C.R. ACAD. SC. PARIS, 1976, t. 282, Serie C, Seiten 65-67; M. DEDIEU et al.: "Etude de transferts intramoléculaires d'hydrogène catalysés par les complexes RuH2L4 et RhHL4"

## Beschreibung

Diese Erfindung betrifft ein neues Verfahren zur Herstellung von Carbonylverbindungen durch katalytische Isomerisierung von Allylalkoholen.

4-Hydroxybutyraldehyd läßt sich z.B. durch säurekatalysierte Hydratisierung von 2,3-Dihydrofuran (Bull.Soc.Chim. France, 1950, 1, 668) oder durch Ozonolyse von 4-Penten-1-ol (J.Am.Soc. 74, (1952), 5324) herstellen. Diese Methoden sind für die Gewinnung von 4-Hydroxibutyraldehyd im technischen Maßstab nicht geeignet.

Man hat Aldehyde auch schon durch katalytische Isomerisierung von Allylalkoholen erhalten. So ist z.B. aus S.G. Davies "Organotransition acetal Chemistry Applications to Organic Synthesis", Pergamon Press, Oxford, 1982, 282-284, bekannt, daß sich Allylalkohol bzw. Methallylalkohol durch Isomerisierung an Metallkomplexverbindung in Propionaldehyd bzw. Isobutyraldehyd umlagern. Bei diesen Verfahren erhält man Reaktionsgemische, aus denen man den Aldehyd nur durch aufwendige Methoden isolieren kann.

Man hat auch schon Rhodiumkatalysatoren für die Isomerisierung von Allylalkoholen zu Aldehyden herangezogen. So wird z.B. Methallylalkohol nach den Angaben in J. Organomet. Chem. 86, (1975), C 17, in Gegenwart von RhH(CO) (PhP)₃ in Isobutyraldehyd umgelagert. Bei dieser Reaktion, die sehr von der Art des Lösungsmittels abhängt, wird eine vollständige Umlagerung bei 70°C und einer Reaktionszeit von 3 Stunden in dem teuren Lösungsmittel Trifluorethanol erzielt. Über die Isolierung des Aldehyds und eine Wiedereinsetzbarkeit des Katalysators werden keine Angaben gemacht. Eingang in die Technik hat auch dieses Verfahren nicht gefunden.

Die Isomerisierung von Allylalkoholen zu Aldehyden wird auch als eine unerwünschte Nebenreaktion bei der Hydroformylierung von Allylalkoholen beobachtet (J. Falbe "New Syntheses with Carbon Monoxide", Springer Verlag, Berlin, 1980, Seiten 107 und 108). Sie tritt insbesondere bei Verwendung von Cobaltkatalysatoren auf, so daß man z.B. bei der Hydroformylierung von Allylalkohol Gemische aus 4-Hydroxybutyraldehyd, 2-Methyl-3-hydroxypropionaldehyd und Propionaldehyd erhält. Die Reindarstellung des 4-Hydroxybutyraldehyds aus diesen Gemischen ist wegen der Neigung des 4-Hydroxybutyraldehyds zu Kondensationen mit hohen Ausbeuteverlusten verbunden. Werden hingegen Katalysatorsysteme aus Rhodiumverbindungen und Phosphinen verwendet, so wird die bei der Hydroformylierung unerwünschte Doppelbindungsisomerisierung zurückgedrängt (Chem. Ztg. 101 (1977), 343-350).

In J. Organometallic Chem. 297, C37-C40 (1985), und C.R. Akad. Sc. Paris, Série C, 65 (1976), wird die diskontinuierliche Isomerisierung von Allylalkoholen mit Hilfe von Rhodium-Phosphin-Komplexen als Katalysator beschrieben. Beide Schriften enthalten keine Angaben über die Abtrennung der Carbonylverbindung vom Homogenkatalysator. Lediglich in der letztgenannten Schrift wird in einem Versuch die gebildete Carbonylverbindung destillativ bei vermindertem Druck vom Katalysator abgetrennt. Diese Maßnahme führt aber zu erheblichen Ausbeuteverlusten, die möglicherweise auf die Inaktivierung des Katalysators unter diesen Bedingungen zurückzuführen sind.

Es wurde nun gefunden, daß man Carbonylverbindungen durch katalytische Isomerisierung von Allylalkoholen, bei der man den Allylalkohol in Gegenwart einer Rhodiumverbindung auf 80 bis 180°C erhitzt, besonders vorteilhaft herstellen kann, wenn man in einem tertiären Phosphin oder Phosphit arbeitet, ein Trägergas durch das Reaktionsgemisch leitet, und die Carbonylverbindung aus dem ausgetragenen Gasgemisch durch Abkühlen abscheidet.

Das neue Verfahren ermöglicht es, Allylalkohole bei nahezu quantitativem Umsatz selektiv in Aldehyde zu überführen, wobei sich die Produktabtrennung als besonders einfach erweist. Dieses vorteilhafte Ergebnis ist überraschend, da bei der Hydroformylierung von Allylalkoholen in Gegenwart von Rhodiumkomplexen eine Umlagerung zu Aldehyden durch den Zusatz von Phosphinen bekanntlich unterdrückt wird.

Nach dem erfindungsgemäßen Verfahren lassen sich z.B. die Carbonylverbindungen der Formel I 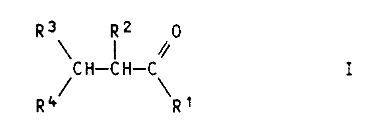
aus den Allylalkoholen der Formel II 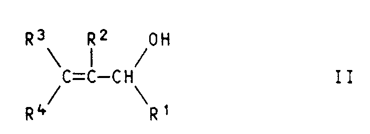
herstellen, wobei in den beiden Formeln R¹ und R⁴ Wasserstoffatome oder Methylgruppen, R² ein Wasserstoffatom, eine Methylgruppe oder eine Carbalkoxygruppe und R³ ein Wasserstoffatom, eine Methylgruppe oder eine Hydroximethylgruppe bedeuten, und R¹ und R⁴ außerdem gemeinsam für ein aliphatisches oder olefinisches Kohlenwasserstoffkettenglied mit 3 bis 6 C-Atomen stehen können.

Allylalkohole der Formel II sind z.B. Allylalkohol, Methallylalkohol, 2-Cyclohexenol, Octa-2,7-dien-1-ol, 3,7-Dimethyl-2,6-octadien-1-ol (Geraniol, Nerol), 3-Methyl-2-buten-1-ol, 2-Buten-1,4-diol, 2-Methyl-2-buten-1,4-diol und 3-Hydroxy-2-methylenbuttersäuremethylester. Das neue Verfahren ist besonders gut für die Herstellung von 4-Hydroxibutanal aus 2-Buten-1,4-diol geeignet.

Als Rhodiumverbindungen kommen z.B. HRh(Ph₃P)₃CO, Rh(acac)(CO)₂, ("acac" = Acetylacetonat), Rhodiumacetat, Rhodiumnitrat, RhCl(Ph₃P)₂CO, Rh(Ph₃P)₃Cl und Rhodiumchlorid in Betracht. Geeignet sind alle Rhodiumverbindungen, die im Reaktionssystem in Lösung gehen und unter den Reaktionsbedingungen Rhodiumhydridkomplexe bilden können.

Als tertiäres Phosphin verwendet man bevorzugt Triphenylphosphin (Ph₃P). Geeignet sind z.B. Phosphine der Formel PAr₃₋ₙRn mit Ar = Aryl, wie Phenyl, R = Alkyl oder Cycloalkyl und n = 0, 1, 2 und 3. Als Phosphite kommen z.B. Verbindungen der Formel P(OAr)₃₋ₙ(OR)ₙ mit Ar = Aryl, wie Phenyl, R = Alkyl und n = 0, 1, 2 oder 3 in Betracht.

Das molare Verhältnis zwischen dem Phosphin bzw. Phosphit zu der Rhodiumverbindung stellt man z.B. auf Werte zwischen 3:1 und 5000:1, wobei der Bereich von 50:1 bis 500:1 bevorzugt ist.

Nach dem Verfahren der Erfindung lagert man den Allylalkohol in dem Gemisch aus dem Phosphin bzw. Phosphit und der Rhodiumverbindung bei Temperaturen von 80 bis 180°C, vorzugsweise 100 bis 150°C um. Dabei verfährt man zweckmäßigerweise so, daß man zunächst das Gemisch aus der Phosphorverbindung und der Rhodiumverbindung auf die gewünschte Reaktionstemperatur erhitzt, wobei sich eine Schmelze bildet, die die Rhodiumverbindung gelöst enthält, und dann den Allylalkohol in die Schmelze einträgt. Gleichzeitig leitet man ein Trägergas durch das Reaktionsgemisch, wodurch die durch Umlagerung entstandene Carbonylverbindung aus dem Reaktionsgemisch entfernt wird. Die Carbonylverbindung, die dabei durch den Trägergasstrom gasförmig ausgetragen wird, isoliert man aus dem Gasstrom durch Abkühlung. Vorzugsweise trägt man den Allylalkohol in dem Maße in die Schmelze ein, in dem die Carbonylverbindung bei möglichst vollständiger Umsetzung entsteht und durch den Gasstrom aus dem Reaktionsgemisch ausgetragen wird. Das Trägergas kann nach der Kondensation und Abtrennung der Carbonylverbindung erneut für eine Einleitung in die Schmelze verwendet und damit im Kreis gefahren werden.

Als Trägergas verwendet man z.B. Stickstoff, Kohlenmonoxid, Methan, Wasserstoff oder Mischungen dieser Gase. Besonders vorteilhafte Ergebnisse werden mit Wasserstoff oder einem Trägergas erzielt, das Wasserstoff enthält. Beispielsweise besteht das Trägergas etwa zu 0,1 bis 100 Vol.% aus Wasserstoff und zu 0 bis 99,9 Vol.% aus Kohlenmonoxid, Stickstoff, Argon oder Methan.

Die durch Abkühlung aus dem Gasstrom abgeschiedene Carbonylverbindung läßt sich, falls erforderlich, durch eine einfache Reindestillation in hoher Reinheit gewinnen.

### Beispiel 1

In einem thermostatisierten Glasgefäß wurden 5,0 g Rhodiumacetat (39,7 Gew.% Rh, 19,3 mMol) sowie 80 g Ph₃P (0,31 Mol) auf 130°C erhitzt. In die Schmelze wurden über eine Glasfritte 100 l/h CO/H₂ (1:1) erhöht und über einen Zeitraum von 7 Stunden insgesamt 356 ml (380 g) 2-Buten-1,4-diol zudosiert (50,9 ml/h). Das ausgetragenen Gas wurde über einen mit Wasser gekühlten Kühler in eine Produktvorlage geführt, die mit Aceton/Trockeneis gekühlt wurde. Es wurden 370 g eines Kondensats isoliert, welches nach gaschromatographischer Analyse 90,5 % 4-Hydroxybutyraldehyd, 2,1 % Butan-1,4-diol und < 0,1 % 2-Buten-1,4-diol enthielt (Umsatz > 99,9 %, Selektivität 90,5 %).

### Beispiel 2

In der in Beispiel 1 beschriebenen Apparatur wurden 1,0 g RhH(CO)(Ph₃P)₃ (1,1 mMol) und 80 g Ph₃P (0,31 Mol) auf 130°C erhitzt und in die Schmelze 164 l/h CO/H₂ (1:1) eingeleitet. Innerhalb von 58 Stunden wurden 1120 ml (1190 g) 2-Buten-1,4-diol zudosiert (19,3 ml/h). Es wurden 1076 g eines Kondensats isoliert, welches nach gaschromatographischer Analyse 77,2 % 4-Hydroxybutyraldehyd, 1,3 % Butan-1,4-diol und 14,2 % 2-Buten-1,4-diol enthielt (Umsatz 85,8 %, Selektivität 90,0 %). Anschließend wurde der Versuch unter Erhöhung des Gasstroms auf 213 l/h CO/H₂ (1:1) fortgesetzt, wobei über 60 Stunden 998 ml (1065 g) 2-Buten-1,4-diol zudosiert wurden (16,7 ml/h). Das Kondensat (1025 g) enthielt nach der gaschromatographischen Analyse 59,2 % 4-Hydroxybutyraldehyd, 0,7 % Butan-1,4-diol und 26,1 % 2-Buten-1,4-diol (Umsatz 73,9 %, Selektivität 80,1 %). Demnach wies der Katalysator auch nach einer Laufzeit von 118 Stunden eine hohe Aktivität auf.

### Beispiel 3

In die nach Beispiel 2 hergestellte Schmelze wurden 193 l/h H₂ eingeleitet. Innerhalb von 20,5 Stunden wurden 399 ml (426 g) 2-Buten-1,4-diol zudosiert (19,5 ml/h). Es wurden 370 g eines Kondensats isoliert, welches nach gaschromatographischer Analyse 72,6 % 4-Hydroxybutyraldehyd, 1,9 % Butan-1,4-diol und 9,2 % 2-Buten-1,4-diol enthielt (Umsatz 90,8 %, Selektitivät 80,0 %).

### Beispiel 4

Der in Beispiel 3 beschriebene Versuch wurde fortgeführt, wobei in die Schmelze 108 l/h H₂/N₂ (1:1) eingeleitet wurde. Innerhalb von 7,5 Stunden wurden 145 ml (157 g) 2-Buten-1,4-diol zudosiert (19,3 ml/h). Es wurden 160 g eines Kondensats isoliert, welches nach gaschromatographischer Analyse 76,5 % 4-Hydroxybutyraldehyd, 1,5 % Butan-1,4-diol und 8,3 % 2-Buten-1,4-diol enthielt (Umsatz 91,7 %, Selektitivät 83,4 %).

### Beispiel 5 (Vergleich)

In einen Destillationskolben mit einem Volumen von 250 ml, der mit einer Siedekapillare sowie einer Vigreux-Kolonne von 1 m Länge ausgerüstet war, wurden 3,0 g RhH(CO)(Ph₃P)₃ (3,3mMol), 50 g Ph₃P (0,19 Mol) und 150 g 2-Buten-1,4-diol gegeben. Bei einem Druck von 1 mm Hg wurde auf 120 bis 125°C erhitzt. Innerhalb kurzer Zeit gingen 50,0 g eines Destillats über, wobei die Kopftemperatur der Kolonne von 40 auf 50°C anstieg. Anschließend konnte keine weitere Destillatbildung beobachtet werden, obwohl erst etwa 1/3 des 2-Buten-1,4-diols umgesetzt waren. Das Destillat enthielt nach gaschromatographischer Analyse 91,4 % 4-Hydroxybutyraldehyd, 0,4 % Butan-1,4-diol und 0,5 % 2-Buten-1,4-diol. Der Versuch zeigt, daß in Abwesenheit eines wasserstoffhaltigen Trägergases die gewünschte Isomerisierung zwar ebenfalls in hoher Selektivität gelingt, der Katalysator jedoch rasch an Aktivität verliert.

### Beispiel 6

In ein thermostatisiertes Glasgefäß wurden 60 g Ph₃P (0,23 Mol) und 3,0 g RhH(CO)(Ph₃P)₃ (3,3 mMol) gegeben. Das Gemisch wurde unter Stickstoff auf 135°C erhitzt. Unter Einleiten von 100 l/h CO/H₂ (1:1) über eine in der Schmelze befindliche Glasfritte wurden 80 g/h Allylalkohol zudosiert. Das ausgetragene Gas wurde über einen mit Wasser gekühlten Kühler in eine Produktvorlage geführt, die mit Aceton/Trockeneis gekühlt wurde. In 30 Minuten fielen 38 g Destillat an, welches nach gaschromatographischer Bestimmung 23 Gew.% Propionaldehyd neben 75 % unumgesetztem Allylalkohol enthielt. Demnach betrug der Umsatz 25 % und die Selektivität zu Propionaldehyd 92 %.

### Beispiel 7

Die Katalysatorschmelze aus Beispiel 1 wurde nach Versuchsende 2 Stunden lang mit 150 l/h CO/H₂ (1:1) bei 130°C begast, um Reste an 2-Buten-1,4-diol und an 4-Hydroxybutyraldehyd zu entfernen. Anschließend wurden bei der gleichen Temperatur und Gasbelastung 20 g/h 2-Cyclohexenol dosiert und nach 1 h 19 g eines Destillats isoliert, welches 82 % 2-Cyclohexenol sowie 2,5 % Cyclohexanon enthielt (Umsatz 18 %, Selektivität 14 %). Daneben entstand in ca. 10 % Ausbeute 3-Cyclohexenol.

### Beispiel 8

In der in Beispiel 1 beschriebenen Apparatur wurden 2,0 g RhH(CO)(Ph₃P)₃ (2,2 mMol) sowie 80 g Ph₃P (0,31 Mol) auf 130°C erhitzt und mit 120 bis 160 l/h H₂/N₂ (20:80) begast. 2-Methyl-2-buten-1,4-diol wurde mit einer Rate von 20 bis 25 ml/h zudosiert. Nach einer Versuchsdauer von 6 Stunden wurden 100 g eines Kondensats isoliert, welches nach gaschromatographischer Bestimmung 65,9 % 3-Methyl-4-hydroxybutyraldehyd, 4,1 % 2-Methyl-4-hydroxybutyraldehyd (jeweils in Form der cyclischen Halbacetale) sowie 30 % 2-Methyl-2-buten-1,4-diol enthielt (Umsatz 70 %, Selektitiväten 94,1 bzw. 5,9 %).

### Beispiel 9

In der in Beispiel 1 beschriebenen Apparatur wurden 5,0 g RhH(CO)(Ph₃P)₃ (5,4 mMol) sowie 80 g Ph₃P (0,31 Mol) auf 130°C erhitzt und mit 195 l/h CO/H₂ (1:1) begast. 3-Hydroxy-2-methylenbuttersäuremethylester wurde mit einer Rate von 21 ml/h zugepumpt. Man erhielt 19,5 ml/h eines Destillats, welches laut gaschromatographischer Analyse folgende Zusammensetzung aufweise: 35,8 % 2-Methyl-3-ketobuttersäuremethylester, 41,3 % 2-Methyl-2-butencarbonsäuremethylester und 19,5 % 3-Hydroxy-2-methylenbuttersäuremethylester. Dies entspricht einem Umsatz von 80,5 % und einer Selektivität von 44,5 %.

### Beispiel 10

Der in Beispiel 9 beschriebene Versuch wurde wiederholt, wobei als Trägergas 196 l/h H₂/N₂ (6:94) eingesetzt wurde. Das Destillat wies folgende Zusammensetzung auf: 72,7 % 2-Methyl-3-ketobuttersäuremethylester, 4,8 % 2-Methyl-2-butencarbonsäuremethylester und 21,7 % 3-Hydroxy-2-methylenbuttersäuremethylester. Dies entspricht einem Umsatz von 78,3 % und einer Selektivität von 92,8 %.

### Beispiel 11

In der Beispiel 1 beschriebenen Apparatur wurden 2,3 g RhH(CO)(Ph₃P)₃ (2,5 mMol) und 65 g Ph₃P (0,25 Mol) auf 130°C erhitzt und mit 90 l/h H₂/N₂ (10:90) begast. 2,7-Octadien-1-ol wurde mit einer Rate von 17 ml/h zugepumpt. Nach 1 Stunde wurden insgesamt 12,6 g Kondensat isoliert, das nach gaschromatographischer Analyse folgende Zusammensetzung aufwies: 49,6 % Octenale, 20,2 % 2,7-Octadien-1-ol sowie 30,2 % Octadien-1-ole. Dies entspricht bei einem Umsatz von 79,8 % einer Selektivität von 62,2 %.

### Beispiel 12

Der in Beispiel 2 beschriebene Versuch wurde wiederholt, wobei als Trägergas 155 l/h Argon eingesetzt und pro Stunde 30 ml 2-Buten-1,4-diol zudosiert wurden. Nach 8,5 h mußte die Zugabe des 2-Buten-1,4-diols für 3,5 h unterbrochen werden, da der Stand der Flüssigphase im Reaktionsgefäß angestiegen war. Nachdem der ursprüngliche Stand wieder erreicht war, wurden erneut 30 ml/h 2-Buten-1,4-diol zudosiert. Da der Stand wieder anstieg, wurde nach 1 h die Dosierung für 2,5 h unterbrochen. Anschließend wurden wiederum für 1 h 30 ml/h 2-Buten-1,4-diol zudosiert. Da kein Produkt mehr ausgetragen wurde, wurde der Versuch nach insgesamt 19,5 h abgebrochen. Insgesamt waren 340 ml (363 g) 2-Buten-1,4-diol zudosiert worden. Der Austrag enthielt 180 g 4-Hydroxybutyraldehyd und 148 g 2-Buten-1,4-diol (Umsatz 59,2 %, Selektivität 83,7 %). Der Versuch zeigt, daß mit einem Wasserstoff-freien Trägergas die gewünschte Isomerisierung ebenfalls in hoher Selektivität gelingt. Allerdings ist die Aktivität des gewünschten Katalysators nach 20 h zurückgegangen.

## Patentansprüche

1. Verfahren zur Herstellung von Carbonylverbindungen durch katalytische Isomerisierung von Allylalkoholen, bei dem man den Allylalkohol in Gegenwart einer Rhodiumverbindung auf 80 bis 180°C erhitzt, dadurch gekennzeichnet, daß man in einem tertiären Phosphin oder Phosphit arbeitet, ein Trägergas durch das Reaktionsgemisch leitet, und die Carbonylverbindung aus dem ausgetragenen Gasgemisch durch Abkühlen abscheidet.

2. Verfahren nach Anspruch 1, dadurch gekennzeichnet, daß man Carbonylverbindungen der Formel 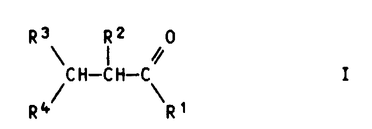 aus Allylalkoholen der Formel 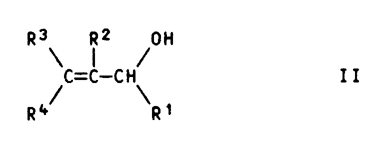 herstellt, wobei R¹ und R⁴ Wasserstoffatome oder Methylgruppen, R² ein Wasserstoffatom, eine Methylgruppe oder eine Carbalkoxygruppe und R³ ein Wasserstoffatom, eine Methylgruppe oder eine Hydroximethylgruppe bedeuten, und R¹ und R⁴ außerdem gemeinsam für ein aliphatisches oder olefinisches Kohlenwasserstoffkettenglied mit 3 bis 6 C-Atomen stehen können.

3. Verfahren nach Anspruch 1, dadurch gekennzeichnet, daß man als Trägergas Wasserstoff oder ein Wasserstoff enthaltendes Trägergas verwendet.

4. Verfahren nach Anspruch 1, dadurch gekennzeichnet, daß man den Allylalkohol in dem Maße in das erhitzte Gemisch aus dem tertiären Phosphin oder Phosphit und der Rhodiumverbindung einträgt, in dem die gebildete Carbonylverbindung mit dem Trägergasstrom aus dem Reaktionsgemisch ausgetragen wird.

5. Verfahren nach Anspruch 1, dadurch gekennzeichnet, daß man 4-Hydroxybutyraldehyd aus 2-Buten-1,4-diol herstellt.

6. Verfahren nach Anspruch 1, dadurch gekennzeichnet, daß man als tertiäres Phosphin eine Verbindung der Formel PAr₃₋ₙRₙ, in der Ar einen Arylrest, R einen Alkyl- oder Cycloalkylrest und n 0, 1, 2 oder 3 bedeuten, und als tertiäres Phosphit eine Verbindung der Formel P(OAr)₃₋ₙ(OR)ₙ, in der Ar einen Arylrest, R einen Alkylrest und n 0, 1, 2 oder 3 bedeuten, verwendet.

7. Verfahren nach Anspruch 1, dadurch gekennzeichnet, daß man als Rhodiumverbindung HRh(Ph₃P)₃CO, Rh(acac)(CO)₂, Rhodiumacetat, Rhodiumnitrat, RhCl(Ph₃P)₂CO, Rh(Ph₃P)₃Cl, Rhodiumchlorid verwendet.

8. Verfahren nach Anspruch 1, dadurch gekennzeichnet, daß man das molare Verhältnis zwischen dem tertiären Phosphin oder Phosphit und der Rhodiumverbindung auf Werte zwischen 3:1 und 5000:1 einstellt.

## Claims

1. A process for the preparation of carbonyl compounds by catalytic isomerisation of allyl alcohols, in which the allyl alcohol is heated to from 80 to 180°C in the presence of a rhodium compound, in which process a tertiary phosphine or phosphite is present, a carrier gas is passed through the reaction mixture, and the carbonyl compound is deposited from the gas mixture, which has been carried out, by cooling.

2. A process as claimed in claim 1, wherein carbonyl compounds of the formula 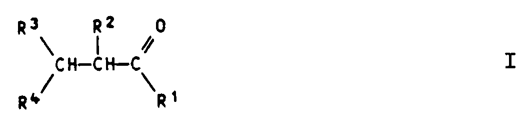 are prepared from allyl alcohols of the formula 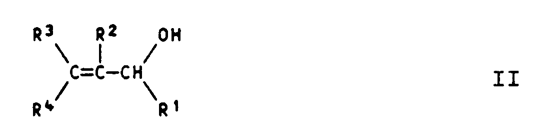 where R¹ and R⁴ are each hydrogen or methyl, R² is hydrogen, methyl or carbalkoxy, and R³ is hydrogen, methyl or hydroxymethyl, and R¹ and R⁴ may additionally together be an aliphatic or olefinic hydrocarbon chain with 3 to 6 carbons.

3. A process as claimed in claim 1, wherein hydrogen or a hydrogen-containing carrier gas is used as carrier gas.

4. A process as claimed in claim 1, wherein the allyl alcohol is fed into the heated mixture of the tertiary phosphine or phosphite and the rhodium compound at the same rate at which the carbonyl compound which is formed is carried out of the reaction mixture with the stream of carrier gas.

5. A process as claimed in claim 1, wherein 4-hydroxybutyraldehyde is prepared from 2-butene-1,4-diol.

6. A process as claimed in claim 1, wherein a compound of the formula PAr₃₋ₙRₙ where Ar is aryl, R is alkyl or cycloalkyl and n is 0, 1, 2 or 3 is used as tertiary phosphine, and a compound of the formula P(OAr)₃₋ₙ(OR)ₙ where Ar is aryl, R is alkyl and n is 0, 1, 2 or 3 is used as tertiary phosphite.

7. A process as claimed in claim 1, wherein HRh(Ph₃P)₂CO, Rh(acac)(CO)₂, rhodium acetate, rhodium nitrate, RhCl(Ph₃P)₂CO, Rh(Ph₃P)₃Cl or rhodium chloride is used as rhodium compound.

8. A process as claimed in claim 1, wherein the molar ratio between the tertiary phosphine or phosphite and the rhodium compound is adjusted to from 3:1 to 5000:1.

## Revendications

1. Procédé de préparation de composés carbonylés par l'isomérisation catalytique d'alcools allyliques, conformément auquel on chauffe l'alcool allylique à 80-180°C en présence d'un composé du rhodium, caractérisé en ce que l'on travaille dans un phosphite ou une phosphine tertiaire, on fait passer un gaz servant de support ou de véhicule à travers le mélange réactionnel et on sépare le composé carbonylé du mélange gazeux sortant par refroidissement.

2. Procédé suivant la revendication 1, caractérisé en ce que l'on prépare des composés carbonylés de la formule 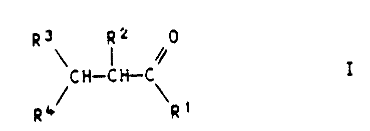 à partir d'alcools allyliques de la formule 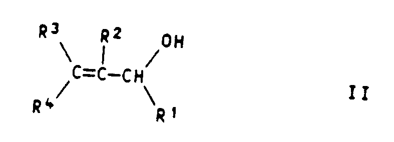 formules dans lesquelles R¹ et R⁴ représentent des atomes d'hydrogène ou des radicaux méthyle, R² représente un atome d'hydrogène, un radical méthyle ou un radical carbalcoxy et R³ représente un atome d'hydrogène, le radical méthyle ou le radical hydroxyméthyle et R¹ et R⁴ peuvent, en outre, représenter ensemble un chaînon hydrocarbure aliphatique ou oléfinique, qui comporte de 3 à 6 atomes de carbone.

3. Procédé suivant la revendication 1, caractérisé en ce que l'on utilise de l'hydrogène ou un gaz contenant de l'hydrogène à titre de gaz servant de véhicule ou de support.

4. Procédé suivant la revendication 1, caractérisé en ce que l'on introduit l'alcool allylique dans le mélange chauffé du phosphite ou de la phosphine tertiaire et du composé de rhodium dans la mesure dans laquelle le composé carbonylé formé sort du mélange réactionnel avec le courant de gaz servant de véhicule ou de support.

5. Procédé suivant la revendication 1, caractérisé en ce que l'on prépare le 4-hydroxybutyraldéhyde à partir du 2-butène-1,4-diol.

6. Procédé suivant la revendication 1, caractérisé en ce qu'à titre de phosphine tertiaire, on utilise un composé de la formule PAr₃₋ₙRₙ, dans laquelle Ar représente un radical aryle, R représente un radical alkyle ou cycloalkyle et n est égal à 0, 1, 2 ou 3 et en ce qu'à titre de phosphite tertiaire, on utilise un composé de la formule P(OAr)₃₋ₙ(OR)ₙ, dans laquelle Ar représente un radical aryle, R représente un radical alkyle et n est égal à 0, 1, 2 ou 3.

7. Procédé suivant la revendication 1, caractérisé en ce que, à titre de composé du rhodium, on utilise les composés des formules HRh(Ph₃P)₃CO, Rh(acac)(CO)₂, l'acétate de rhodium, le nitrate de rhodium, les composés des formules RhCl(Ph₃P)₂CO, Rh(Ph₃P)₃Cl, le chlorure de rhodium.

8. Procédé suivant la revendication 1, caractérisé en ce que l'on règle le rapport molaire entre le phosphite ou la phosphine tertiaire et le composé du rhodium à des valeurs qui fluctuent de 3:1 à 5000:1.
